# EUROPEAN PATENT APPLICATION

(11) **EP 2 008 605 A1**
(43) Date of publication of application: **31.12.2008**
(21) Application number: 07110967.2
(22) Date of filing: 25.06.2007
(51) Int. Cl.: A61B 19/00

(54) **A hybrid manual-robotic system for controlling the position of an instrument**

(71) Applicant: UNIVERSITE CATHOLIQUE DE LOUVAIN, 1348 Louvain la Neuve (BE); Clinique Universitaires Saint-Luc, 1200 Bruxelles (BE)
(72) Inventor: The designation of the inventor has not yet been filed
(74) Representative: Brants, Johan P.E.

(57) **Abstract**

The present invention relates to a hybrid manual-robotic system for supporting and moving a surgical instrument having an active main structure (100) that comprises: a) a base (2) for attachment to an operating table having an operating surface (150); b) a hexahedral-shape frame (3), which shape is formed from a first (5, 5') and second (4, 4') pair of opposing parallelograms and opposing proximal (6') and distal (6) rectangles, which frame (3) is formed by at least seven links (7, 8, 9, 10, 11, 12, 13, 22, 23) connected by revolute joints (14, 15, 16, 17, 18, 19, 20, 26, 27, 109); c) whereby a pair of parallel base links (7, 13) of the proximal rectangle (6') is coupled to the base (2) and are configured to lie essentially perpendicular to the plane of the operating surface (150); d) whereby said revolute joints are configured to allow the hexahedral frame (3) to freely adopt a cube, or parallelepiped shape restricted by said perpendicular coupling; and e) whereby the distal rectangle (6) is coupled through a transmission means (200) to said instrument.

## Description

### FIELD OF THE INVENTION

The present invention relates to a hybrid manual-robotic system that supports and moves remotely an instrument in response either to manual intervention, to an electronic input device or both.

### DESCRIPTION OF RELATED ART

Surgical procedures have changed dramatically during the past thirty years. The traditional approaches were opened up new ways by the emergence of the endoscopic technique, which strongly reduces the per- and post-operative traumatism, and the risks of infection. In addition, the pain is decreased as well as the size of scars.

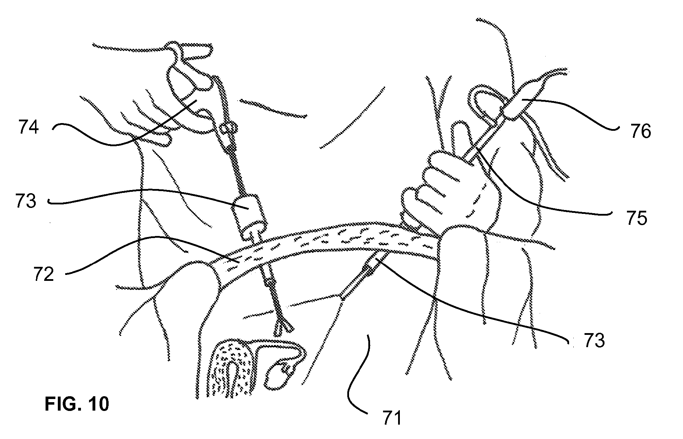

However, if this new technique offers many advantages to the patient, it complicates the task of the medical team appreciably. Indeed, to be able to visualize the interior of the distended abdominal cavity **71**, a laparoscope **75** is introduced in it through a cannula **73**, often called trocar, and a camera **76** fixed at the proximal end of the laparoscope **75** sends the images to a monitor. These basic principles of laparoscopic surgery are illustrated in **Fig. 10**.

The surgeon needing his two hands to carry out the intervention, the handling of the camera is then entrusted to another surgeon, or more often to a little tested assistant. The surgeon consequently does not have direct control of the movements of the camera any more, which makes the surgical gestures more difficult to realize. Communication problems may also occur and induce unwanted motions, and after a while, the image often becomes unstable because of tremors.

Furthermore, whereas certain traditional laparotomic procedures can be achieved by a surgeon alone, the laparoscopic way requires one more person to hold the camera and to leave the surgeon's two hands free.

Passive laparoscope holders consist of several bars connected with lockable joints. They are attached on the operating table rail, and their tip holds the endoscope. The surgeon can grasp the holder and move it to a suitable position, after releasing the joints.

Some of them comprise pneumatically controlled ball-joints, which can be released by pressing a button or a foot pedal. Examples are: Unitrac - Endofreeze (Aesculap, Tuttlingen, Germany), Leonard Arm (Leonard Medical Inc., Huntingdon Valley, USA) and Endoboy (Geyser-Endobloc S.A., Coudes, France). Others are mechanically locked using knobs or adjustable friction, like the Martin Arm (Karl Storz, Tuttlingen, Germany), the Low Profile Scope Holder (Kronner, Roseburg, USA) and the PASSIST (Academic Medical Centre, Amsterdam, Netherlands).

These devices offer a better image stability, never get tired, give the control of the motions back to the surgeon, and allow solo surgery during a part or entirety of certain procedures. However, at least one hand is needed to move the camera, so that the surgeon must frequently release an instrument, and therefore remove it from the cannula to avoid any risk of accidental wound.

Active scope holders overcome this limitation. Some of their joints are actuated by electrical motors, so that the surgeon only has to tell the robot where to go. Various means are used to control these electromechanical arms without having to release an instrument : voice control for Aesop (Computer Motion Inc, Goleta, USA), head control with a helmet for EndoAssist (Armstrong Healthcare Inc., Acton, USA), finger control with a remote control placed inside the surgeon's glove for LapMan (Medsys SA, Gembloux, Belgium) or a joystick placed on the instrument for Naviot (Hitachi Ltd., Tokyo, Japan) and FIPS Endoarm (Nuclear Research Institute, Karlsruhe, Germany), foot control for Laparocision (GMP Surgical Solutions Inc., Lauderdale, USA), instrument tracking for LER (TIMC, Grenoble, France).

Although all of these devices function properly, these positioners suffer from several drawbacks. Firstly, the motions of these robots are slow and quite basic (only left-right, up-down and in-out for most of them) and limited in range, mainly due to the electromechanical structure and the control method or device.

Moreover, some of them are placed on the ground, next to the operating table, like EndoAssist and LapMan. These devices must be heavy and quite large, to guarantee stability. As a result, they are bulky in a place where space is required for the surgeons and nurses. Aesop is a bit smaller and attached to the table rail. But because of its weight, it must be carried on a dedicated mobile station and the procedure to secure it on the table and remove it from the mobile base is rather long and constraining. LER is small and lightweight, and is placed directly on the patient's abdomen. This is very convenient and does not constraint the placement of the surgeons, but may restrict the placement of other cannulas.

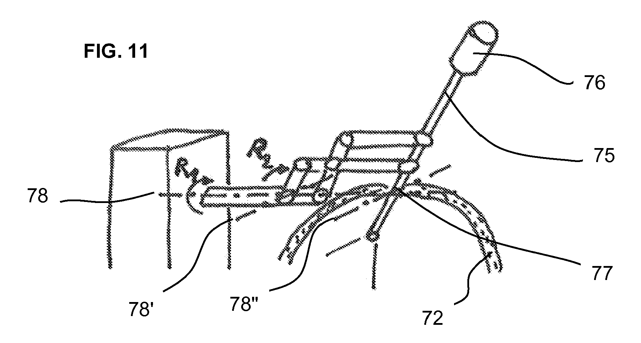

Furthermore, EndoAssist, LapMan, FIPS Endoarm, Laparocision and LER use an architecture that has a mechanical center or axis of rotation, like the example shown in Fig. **11**. The presence of this center **77** or axes of rotation **78, 78', 78"**, intrinsic to the structure, imposes a perfect alignment between the robot and the incision, which is the natural stationary point for the laparoscope **75** in the abdominal wall **72.** A wrong alignment may lead to wrong motions of the laparoscope **75**, or even be dangerous for the patient. Setup requires therefore several minutes just to adjust the robot to the patient and align it properly. And if the operating table is readjusted during the procedure with a robot placed on the ground (EndoAssist, LapMan), the robot must then be realigned.

Finally, Aesop, EndoAssist and LapMan grasp the laparoscope just below the camera, and require a large motion of the arm to perform a simple zoom (in or out) motion. FIPS Endoarm uses a slider mechanism that avoids this arm motion, but the range of motion of this mechanism is quite short. Naviot uses built-in camera and endoscope with optical zoom dedicated to thoracoscopy, which restricts its field of use.

For all these reasons, it would be desirable to provide an active laparoscope holder that combines all of the advantages of the different existing systems. It should be small and easy to carry, have the largest workspace possible with small motions of the structure. The zoom motion should be obtained with no motion of most of the parts of the device. One should be able to position it independently of the incision, so as to let all the team members choose their own placement without additional constraint. It should also be quick to install, requiring less possible adjustments to the patient and to the type of procedure.

### SUMMARY OF THE INVENTION

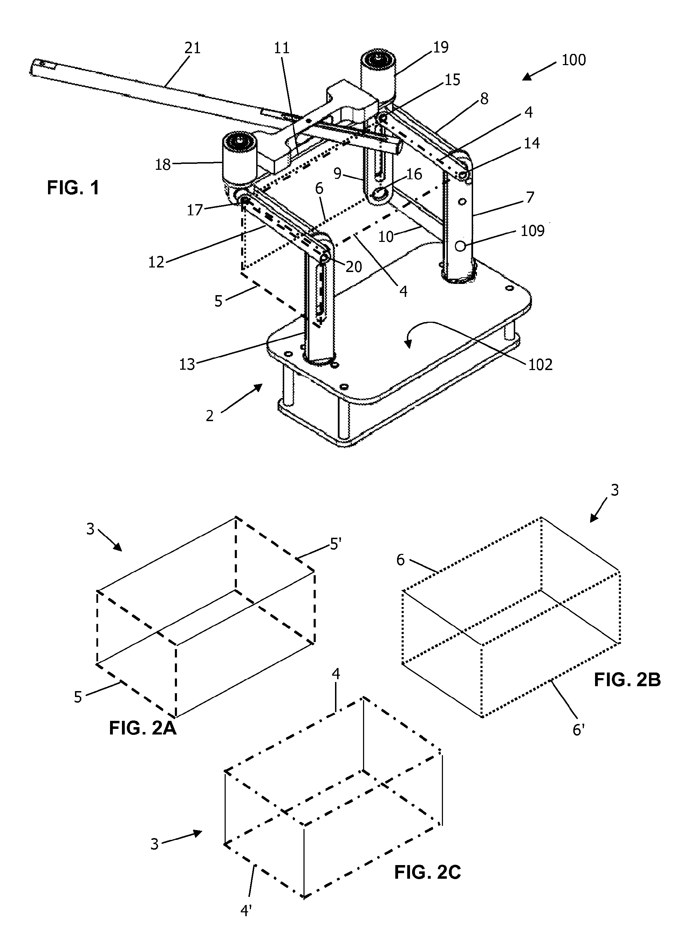

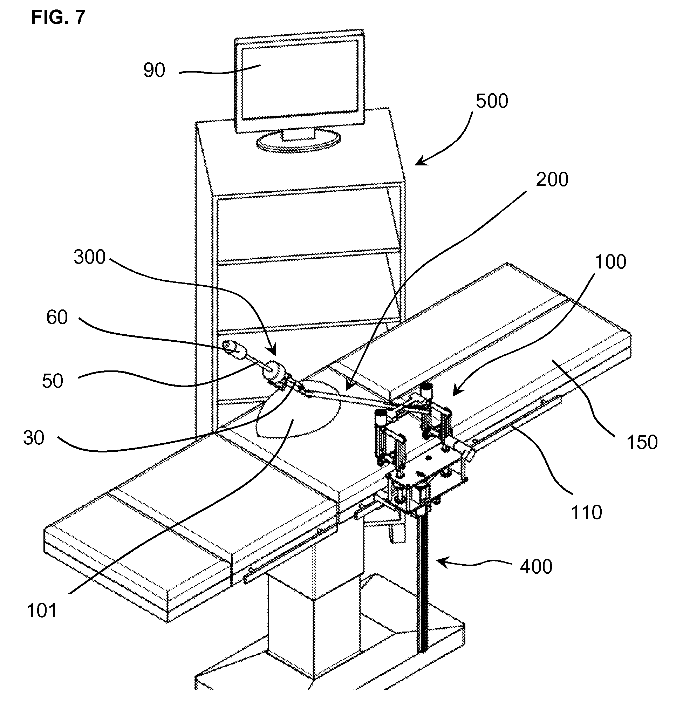

The present invention relates to a hybrid manual-robotic system that supports and moves remotely a surgical instrument in response either to manual intervention, to an electronic input device or both. It is based on an active main structure **100** as shown in **FIGs. 1** and **2**, which comprises:
a) a base **2** for attachment to the operating table having an operating surface **150** (**FIG. 7**),
b) a hexahedral-shape frame **3**, which shape is formed from two pairs of opposing parallelograms **4, 4', 5, 5'** and opposing proximal **6'** and distal **6** rectangles, which frame is formed by at least seven links e.g. **7, 8, 9, 10, 11, 12, 13, 22, 23** connected by revolute joints **14, 15, 16, 17, 18, 19, 20, 26, 27,109**
c) whereby a pair of parallel links **7, 13** (base links) of the proximal rectangle is coupled to the base **2** and are configured such that their longitudinal axes are essentially perpendicular to the plane of the operating surface **150**,
d) whereby said revolute joints allow the hexahedral frame **3** to freely adopt a cube, or parallelepiped restricted by said perpendicular coupling,
e) whereby the distal rectangle **6** is coupled through a transmission arm **21** to said instrument.

The active main structure **100** allows the instrument to swivel around an incision in the abdominal wall, with a freedom of movement that describes a dome parallel to the plane of the patient. Achieving a domed movement path is particularly suited to an instrument such as a laparoscope that enters at a point of incision, and is to be swiveled around said incision while remaining at the same depth below the incision.

The hybrid manual-robotic system may also comprise additional components as shown in **FIG. 7**, including a local zoom device **300**, which is adapted to hold a surgical instrument, such as a laparoscope **50**. The local zoom device **300** is actuated to rotate the instrument around its longitudinal axis, and to advance it and move back in the cannula **30** without any motion of the rest of the system, unlike most of the existing devices presented here before. The local zoom device is linked by the transmission means **200**, to the active main structure **100**,

The unit formed by the local zoom device **300**, the transmission means **200** and the active main structure **100** is called the robotic device **600.** The system may further include a computer, called the central control unit, which among other tasks controls the movement of the local zoom device **300** and the main active structure **100** in response to input signals from a control device.

The hybrid manual-robotic system may be carried by a cart, called the rolling base **500**, which can be wheeled to and from the operating table **10**. At the start of the surgical procedure, hybrid manual-robotic system may be withdrawn from the rolling base **500**, and then mounted onto the lateral table rail **110** by an adjustable clamping mechanism, called the clamping mechanism **400.** The clamping mechanism **400** can contain a lockable slider that allows the robotic device **600** to be raised or lowered relative to the patient and adjusted to the height/level of the incision.

The transmission means **200** is rigid, but may contain locked joints that can be released to move the local zoom device **300** above the patient, in order to place it appropriately near the incision at the beginning of the procedure. This allows the surgeon to choose a suitable position for the main active structure **100** independently of the position of the incision and the procedure, which is impossible with the existing devices listed above.

The local zoom device **300** and the transmission means **200** can be removed from the active main structure **100** at the end of the surgical procedure, to be decontaminated by a sterilization process. The active main structure **100** may be encapsulated by a disposable protective bag, called the sterile bag, which forms a shield between the end-effector of the active main structure **100** and the transmission means **200**. The sterile bag can be removed after the procedure, and prevents the main active structure to be contaminated, so that the system can be reused without having to clean and sterilize the latter.

One embodiment of the present invention is a hybrid manual-robotic system for supporting and moving a surgical instrument having active main structure (100) that comprises:
a) a base (2) for attachment to an operating table having an operating surface (150),
b) a hexahedral-shape frame (3), which shape is formed from
   - a first (5, 5') and second (4, 4') pair of opposing parallelograms and
   - opposing proximal (6') and distal (6) rectangles, which frame (3) is formed by at least seven links (7, 8, 9, 10, 11, 12, 13, 22, 23) connected by revolute joints (14, 15, 16, 17, 18, 19, 20, 26, 27, 109),
c) whereby a pair of parallel base links (7, 13) of the proximal rectangle (6') is coupled to the base (2) and are configured to lie essentially perpendicular to the plane of the operating surface (150),
d) whereby said revolute joints are configured to allow the hexahedral frame (3) to freely adopt a cube, or parallelepiped restricted by said perpendicular coupling, and
e) whereby the distal rectangle (6) is coupled through a transmission means (200) to said instrument.

One embodiment of the present invention is a system as described above, wherein at least one joint of the first (5, 5') pair of opposing parallelograms and at least one joint of the second (4, 4') pair of opposing parallelograms are actuated.

Another embodiment of the present invention is a system as described above, wherein
- the first pair of parallelograms (5, 5') is defined as the pair each comprising one link that is a base link (7, 13) of the proximal rectangle (6'), and
- the first pair of parallelograms are connected by a link of the distal rectangle (6) that is defined as an effector link (11) to which the transmission arm is coupled.

Another embodiment of the present invention is a system as described above, wherein said effector link (11) is an uppermost link of the proximal rectangle (6').

Another embodiment of the present invention is a system as described above, wherein the length of the effector link (11) is greater than the length of the FPs links (8, 10, 12, 23) that are joined to the base links (7, 13).

Another embodiment of the present invention is a system as described above, equipped with a static balancing mechanism (121) configured to passively maintain the position of the instrument after movement.

Another embodiment of the present invention is a system as described above, wherein the transmission means (200) comprises an articulated arm (21) that can be rigidly locked for transmitting movements of the main active structure over the distance of the articulated arm (21) to the instrument.

Another embodiment of the present invention is a system as described above, wherein the transmission means further comprises a fixing mechanism (201) that couples the distal rectangle to the articulated arm (21).

Another embodiment of the present invention is a system as described above, wherein said coupling is a lockable joint configured to allow the articulated arm (21) to:
- pivot parallel to the axis of the operating surface (150) relative to the distal rectangle (6), and
- translate along at least part of its longitudinal axis relative to the distal rectangle (6).

Another embodiment of the present invention is a system as described above, wherein the transmission means further comprises a quick clamping mechanism (202) that couples the instrument to the distal end of the articulated arm (21).

Another embodiment of the present invention is a system as described above, wherein said coupling is a lockable revolute joint configured to allow the quick clamping mechanism (202) to pivot parallel to the axis of the operating surface (150) relative to the articulated arm (21).

Another embodiment of the present invention is a system as described above, wherein the instrument is a laparoscope attached to the quick clamping mechanism (202) via a local zoom device (300).

Another embodiment of the present invention is a system as described above, wherein the base comprises a table clamping mechanism (400) for attachment to a lateral table rail (110) of the operating surface.

### FIGURE LEGENDS

**FIG. 1** Three dimensional drawing of an active main structure of the invention, where the parallelograms and rectangles forming the hexahedral-shape frame are indicated as dashed lines.
**FIGs. 2A to C** Three dimensional drawing of the hexahedral-shape frame, where the first pair of parallelograms (FPs) are indicated as dashed lines (FIG. 2A); where the proximal and distal rectangles are indicated as dashed lines (FIG 2B); and where the second pair of parallelograms (SPs) are indicated as dashed lines (FIG. 2C).
**FIG. 3** Three dimensional view of an active main structure, transmission means and instrument of the invention, where first pair of parallelograms (FPs) are indicated as dashed lines.
**FIG. 4** Alternative three dimensional view of an active main structure, transmission means and instrument of the invention, where first (FPs) pair of parallelograms and a second parallelogram (SP) are indicated as dashed lines.
**FIG. 5** Alternative three dimensional drawing of an active main structure where the base is depicted in close view.
**FIG. 6** Alternative three dimensional drawing of the active main structure, transmission means and instrument of the invention, where a second parallelogram (SP) is indicated as a dashed line.
**FIG. 7** Three dimensional drawing showing an overview of possible components in the system of the invention.
**FIG. 8** Three dimensional drawing showing a detail of the transmission means of the system.
**FIG. 9** Three dimensional drawing showing a detail of the table clamping mechanism.
**FIG. 10** Diagram illustrating the basic principles of laparoscopic surgery.
**FIG. 11** Diagram showing parallelogram design with stationary point which requires a perfect alignment between the two revolution axes and the stationary point in the abdominal wall.

### DETAILED DESCRIPTION OF THE INVENTION

Unless defined otherwise, all technical and scientific terms used herein have the same meaning as is commonly understood by one of skill in the art. All publications referenced herein are incorporated by reference thereto. All United States patents and patent applications referenced herein are incorporated by reference herein in their entirety including the drawings.

The articles "a" and "an" may be used herein to refer to one or to more than one, *i.e*. to at least one of the grammatical object of the article. By way of example, "a base" means one base or more than one base.

The recitation of numerical ranges by endpoints includes all integer numbers and, where appropriate, fractions subsumed within that range (e.g. 1 to 5 can include 1, 2, 3, 4 when referring to, for example, a number of links, and can also include 1.5, 2, 2.75 and 3.80, when referring to, for example, measurements). The recitation of end points also includes the end point values themselves (e.g. from 1.0 to 5.0 includes both 1.0 and 5.0)

The present invention relates to a hybrid manual-robotic system that supports and moves remotely a surgical instrument in response either to manual intervention, to an electronic input device or both. The inventors have found that an adjustable frame, based on an arrangement of two pairs of parallelograms and one pair of rectangles disposed to form a parallelogram-faced hexahedral provides the instrument attached thereto a freedom of movement that describes a dome parallel to the plane of the patient. Achieving a domed movement path is particularly suited to an instrument such as a laparoscope that enters at a point of incision, and is to be swiveled around said incision while remaining at the same depth below the incision. It does not require accurate alignment of the moving parts with respect to the patient, unlike systems of the art. The positioned instrument is held steady by the system within the region of the dome without the need for assisted locking.

Because the parallelograms contain a plurality of revolute joints, the placement of the instrument is controllable by rotary servos and encoders. If required, the instrument can also be moved manually without any special adaptation.

Reference is made in the description below to the drawings which exemplify particular embodiments of the invention; they are not at all intended to be limiting. The skilled person may adapt the invention and substitute components and features according to the common practices of the person skilled in the art.

With reference to **FIGs. 1** to **3**, the present invention relates to a hybrid manual-robotic system to move and support an instrument comprises an active main structure **100** which comprises:
a) a base **2** for attachment to the operating table having an operating surface **150** (**FIG. 7**),
b) a hexahedral-shape frame **3**, which shape is formed from two pairs of opposing parallelograms **4, 4', 5, 5'** and opposing proximal **6'** and distal **6** rectangles, which frame is formed by at least seven links e.g. **7, 8, 9, 10, 11, 12, 13, 22, 23** connected by revolute joints **14, 15, 16, 17, 18, 19, 20, 26, 27, 109,**
c) whereby a pair of parallel links **7, 13** (base links) of the proximal rectangle is coupled to the base **2**, and are configured such that their longitudinal axes are essentially perpendicular to the plane of the operating surface **150**,
d) whereby said revolute joints are configured to allow the hexahedral frame to freely adopt a cube, or parallelepiped restricted by said perpendicular coupling (item d),
e) whereby the distal rectangle **6** is coupled through a transmission arm **21** to the instrument.

The hexahedral frame that forms the basis of the active main structure **100** is shown in isolation in **FIGs. 2A, 2B** and **2C**. The hexahedral frame is the geometric shape formed essentially from two pairs of parallelograms **4, 4', 5, 5'** and one pair of rectangles **6, 6',** each pair arranged on opposing faces of the hexahedron. For clarity **FIGs. 2A** to **2C** indicate the pairs of parallelograms on separate hexahedrons while **FIG. 1** indicates only one of each pair of hexahedrons **4, 5** and rectangle **6.** As used herein, the term parallelogram refers to the geometric shape having two pairs of opposite parallel lines. A rectangle falls under this definition. A rectangle is a quadrilateral where all four of its internal angles are right angles and so it has two pairs of parallel sides. The lengths of two opposing sides can be the same (square) or different (oblong).

The geometric structure formed by the hexahedral frame may be a cube when the parallelograms become rectangular, or a parallelepiped when an internal angle of any one of the parallelograms is other than 90 deg. The internal angles adopted by the parallelograms can change depending on the position of the instrument, while the internal angles adopted by the rectangles remains at 90 deg.

Two pairs of opposing parallelograms can be defined as the first and second pairs of parallelograms. The first pair of parallelograms **5, 5'** of the hexahedral frame, is defined as the opposing pair where each parallelogram **5, 5'** comprises one link that is a base link **7, 13** of the proximal rectangle **6'**. Each parallelogram **5, 5'** of the first pair is known as a "FP" herein. The second pair of parallelograms **4, 4'** of the hexahedral frame, is the other opposing pair of parallelogram **4, 4'**. Each parallelogram **4, 4'** of the second pair is known as is known as a "SP" herein.

The position, orientation and range of movement possible by the hexahedral frame is constrained because a pair parallel links (base links) of the proximal rectangle is coupled to the base, preferably pivotally coupled, essentially perpendicular to the plane of the operating surface.

This has the effect that the first parallelograms lie such that their planes are essentially perpendicular to the operating surface when base is mounted on the operating table, regardless of the shape the hexahedral frame adopts.

This also has the effect that the rectangles **6, 6'** of the hexahedral frames also lie such that their planes are essentially perpendicular to the operating surface when base is mounted on the operating table, regardless of the shape the hexahedral frame adopts.

The position and orientation of the second parallelograms **4, 4'** of the hexahedral frames, lie such that their planes tilt with respect to the operating surface when base is mounted on the operating table.

The terms "distal" and "proximal" are used through the specification, and are terms generally understood in the field to mean towards (proximal) or away (distal) from the surgeon's end of the apparatus. Thus, "proximal" means towards the surgeons end and, therefore, away from the patient's end. Conversely, "distal" means towards the patient's end and, therefore, away from the surgeon's end.

### Links

The hexahedral frame is formed using a plurality of mechanical links **7, 8, 9, 10, 11, 12, 13, 22, 23** which form the edges of the active main structure. The skilled person will understand that a suitable hexahedral frame does not require the presence of a link along every edge of the frame *i*.*e*. along all 12 edges. The absence of one link in a parallelogram or rectangle can be compensated by its presence in the opposing parallelogram or rectangle without compromising the integrity of the frame geometry. The inventors have found that a minimum of 7 links is sufficient, though there may be 8, 9, 10, or 11 links for additional stability to support a heavy instrument, the twelfth link being formed by the base **2**. Preferably the number of links is 9 which provides a compromise between loading bearing, cost of construction and performance of the mechanism.

According to one aspect of the invention, a link is formed from one or more rigid bars connected in parallel. The bars can be solid or hollow (e.g. hollow tubes for lightness). During operation by a surgeon, the forces and torques are applied to the links, which come from, amongst others: gravity which is related to the combination of joint angles at that instant in time, acceleration, and the operator induced forces and torques. The links should be formed from a stiff material having requisite tensile and compression characteristics to support the instrument and to withstand forces and torques. A link maybe formed of aluminium which has suitable strength and lightness. Alternatively a link may be formed from a thin-walled tube made of a woven carbon fiber-epoxy composite material such as Toray T700 that provides a near-zero coefficient of thermal expansion, high stiffness and low density.

The arrangement of the seven links can be readily determined by the skilled person with a knowledge of mechanics and/or geometry to form a robust active structure. Preferably, the at least seven links comprise:
i) the base links **7, 13** of item d) which form an incomplete proximal rectangle **6'** (2 links),
ii) three links **8, 9, 10** which, with one base link of item i) **7**, form a complete parallelogram,
iii) one link **12**, which with the other base link of item i) **13**, forms an incomplete parallelogram opposite the parallelogram of item iii),
iv) one link **11**, which with one link of item ii) **9** forms an incomplete distal rectangle **6**.

The hexahedral frame is articulated so that it can move within a cube or a sheared cube (parallelepiped) configuration. The links of the hexahedral frame are linked by joints which are preferably revolute joints. The joints are preferably located in the vicinity of the corners of the frame, and which allow movement of the frame within a cube or a sheared cube (parallelepiped) configuration. The shape of the parallelepiped is constrained because the base links **7, 13** are pivotally coupled the base essentially perpendicular to the operating surface. The longitudinal axis of each base link **7, 13** is thus perpendicular to the operating surface. This means both rectangles **6, 6'** have parallel faces that remain perpendicular to the operating surface when the frame is moved around its joints.

The coupling of the base links **7, 13** to the base **2** does not permit a translational or angular movement by the base links **7, 13** relative to the base **2**. However, it may permit a revolute (rotational, but not pivotal or translational) movement of each base link relative to the base.

As the frame is moved, any given point on the distal rectangle link **11**, called the effector link (see below) will move within a surface described by a dome. An instrument coupled to the distal rectangle will have limited movement within said dome. The instrument is preferably coupled to the distal rectangle via a(n) (effector) link **11**, using one or more linkages. The linkage not only transmits the dome-like movement of the frame, but also supports the weight of the instrument.

### First pair of parallelograms (FPs)

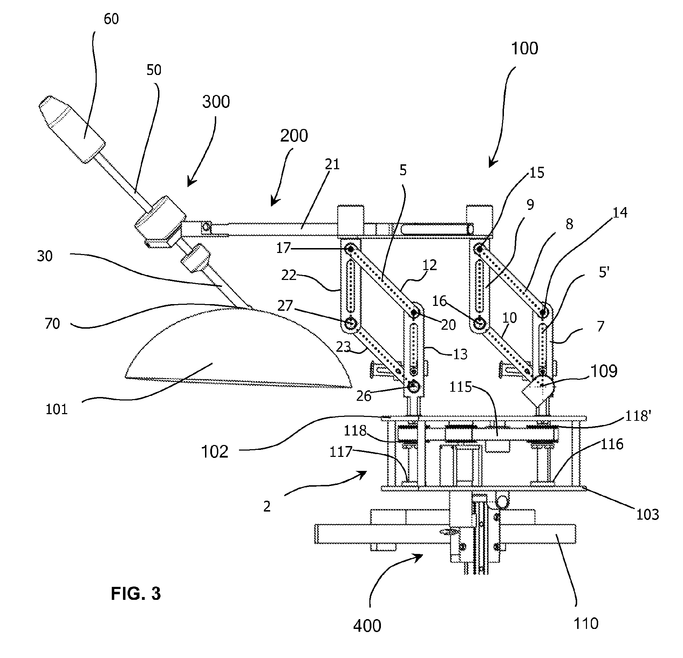

The active main structure 100 comprises a first pair of opposing parallelograms (two FPs); one of the pair is known as a first FP and the other in the pair is known as the second FP. The first FP **5'** formed from four links **7, 8, 9, 10 (****FiGs. 1** and **3**), the links being connected by four revolute joints **14, 15, 16, 109**. The axis of revolution of each joint is normal to the plane of the first FP. When any of the four revolute joints **14, 15, 16, 109** is rotated (e.g. actuated), the shape of the FP changes.

A second FP **5** is disposed on the face of the hexahedral frame opposite the first FP **5'**. It may be formed from 2 or more (*e.g.* 3 or 4) links **13, 14, 22, 23**, the links being connected by revolute joints **17, 20, 26, 27**. The axis of revolution of each joint **17, 20, 26, 27** is normal to the plane of the second FP **5**. When any of the four revolute joints **17, 20, 26, 27** is rotated (e.g. actuated), the shape of the FP changes.

The FPs cause the effector link **11**, present in the distal rectangle **6** which joins the first FP **5'** to the second FP **5**, to follow a circular trajectory, whose radius is determined by the length of the upper FP links **8, 12** and lower FP links **10, 23**.

### Proximal and distal rectangles

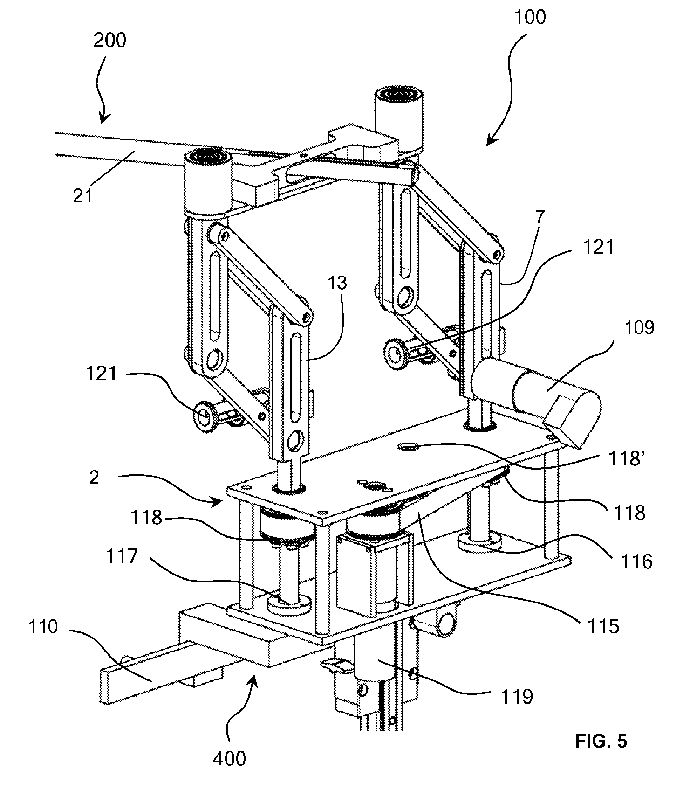

The active main structure **100** also comprises a proximal rectangle **6'** comprising two or more links, whereby at least two links are opposing links that belong to the first **5'** and second **5** FPs, and which are also the base links **7, 13 (****FIGs. 1** and **3**). Said base links **7, 13** are coupled to the base **2** such that the longitudinal axes of the base links are perpendicular to the operating surface **150**. According to a preferred embodiment of the invention, the base links **7, 13** are revolutely coupled to the base **2**, such that said links rotate about their respective longitudinal axes. The base links **7, 13** may be coupled to the base **2** using any coupling which provides the required orientation and revolute motion. One example of a suitable revolute coupling is shown **FIG. 5**, whereby the base links **7, 13** are extended in the longitudinal direction, pass though the top plate **102** of the base 2 and couple to a lower plate **103** in the base **2** in a revolute joint **116, 117**. In one embodiment of the invention, the base links **7, 13** are extended in the longitudinal direction and are each coupled to the base *via* the end of the extended part such that each base link can rotate around its longitudinal axis relative to the base.

The edges of the proximal **6'** and distal **6** rectangles perpendicular to the base links **7, 13** are preferably of a length such that each of FPs **5, 5'** can rotate around the base links **7, 13** about 360 deg without colliding with each other. Thus, the length of the link **11** joining the first FP **5'** to the second FP **5** is preferably greater than the length of the FPs links **8, 10, 12, 23** that are joined to the base links **7, 13.**

### Effector link

A distal rectangle **6** is disposed on the face of the hexahedral frame opposite the first rectangle **6'.** It may be formed from 2 or more (*e.g.* 3 or 4) links, whereby one link **9** belongs to the first FP, and a second link **11** - an effector link - joins the first FP **5'** to the second FP **5**. Said effector link **11** may be connected by revolute joints **18, 19** to the first and second FPs; the axis of revolution of each joint **18, 19** of said effector link **11** is parallel to the longitudinal axes of the base links **7**, **13.** The effector link **11** is preferably the uppermost link of the proximal rectangle (6'). The effector link **11** allows each FPs to rotate in concert, 360 deg around their respective base links **7, 13**. During such rotation, the longitudinal axis of the effector link **11** remains parallel to the plane of the operating table. The effector link **11**, therefore, is the preferred site of attachment of a linkage **21** to the instrument. The effector link **11** preferably joins the first **5'** and second **5** FPs across the top of the distal rectangle **6.**

### Second pair of parallelograms (SPs)

The active main structure **100** further comprises a second pair of opposing parallelograms (two SPs); one of the pair is known as the upper SP **4** and the other in the pair is known as the lower SP **4'**. The second pair of parallelograms (SPs) **4, 4'** are the remaining parallelograms formed when the hexahedron is disposed with the above mentioned FPs and rectangles. Each SP **4, 4'** is formed from the links used to construct the FPs and the rectangles. According to one aspect of the invention, the upper SP **4** comprises the effector link **11,** and two upper links **8, 12** of each SP. According to another aspect of the invention, the lower SP **4'** comprises at least the two lower links **10, 23** of each FP.

### Actuated joints

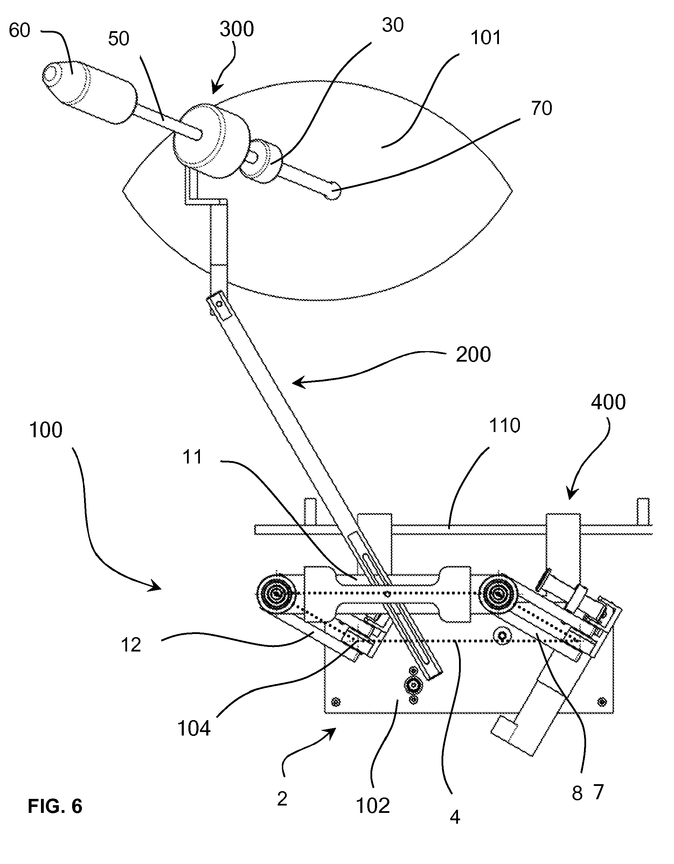

**FIG. 3****,** **FIG. 4****,** **FIG. 5** and **FIG. 6**, show alternative views and configurations of the architecture of the active main structure **100**. The FPs **5, 5'** are highlighted in **FIG. 3** having four links (**7, 8, 9, 10** and **12, 13, 22, 23**) per FP whereby one link in each FP is a base link **7,13**. Base links **7, 13** are attached to the base **2** through revolute joints. Also indicated in **FIG. 3** is an actuated joint **109** that can move (*i*.*e*. change the shape of) the first FP through a range of movement using motorized control such as a servo or stepper motor. There is preferably one actuated joint present in the FPs, which actuated joint is preferably the revolute joint that connects one base link **7, 13** with another link of the same FP **9, 10, 12, 23**. In the case of **FIG. 6**, the actuated joint is that which joins base link **7** a FP link **10**.

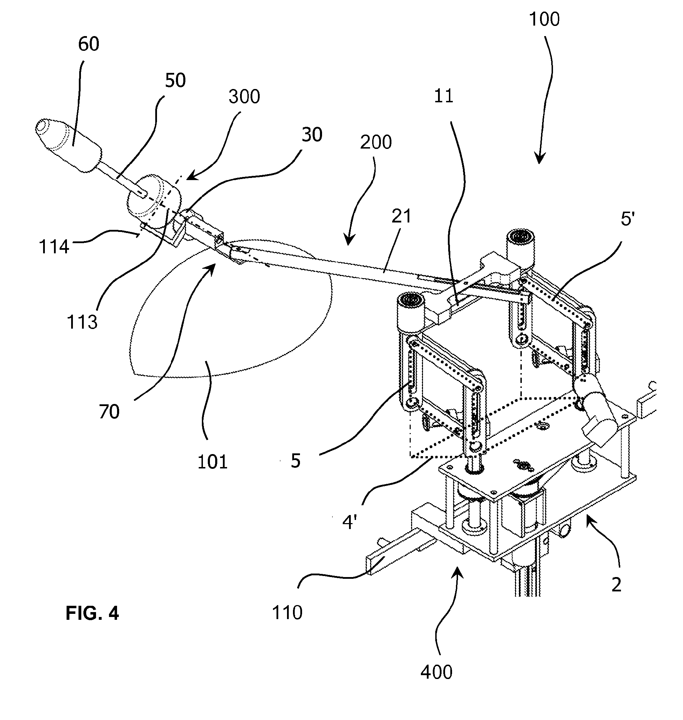

The combination of these FPs **5** and **5'**, seen in **FIG. 4****,** forces the effector link **11** to move on the surface of a dome, without making any rotation with respect to the base **2**. When an instrument or a laparoscope **50** is connected to the effector link **11** by a passive adjustable arm **22**, whose extremity has two perpendicular free revolute joints **113** and **114**, the motion of the main active structure **100** allows a laparoscope **50** to swivel around the incision **70** in every direction, just as a surgeon's hand would do if he held the laparoscope **50** directly.

The presence of two FPs **5, 5'** improves the rigidity of the structure, though only one FP needs to be actuated. Rigidity may be assisted by a timing belt **115,** depicted in **FIGs. 3** and **5**, which connects the two revolute joints **116** and **117** of the base links **13, 7** through two pulleys **118**. Tension in the belt may be adjusted by an idler pulley **118'**.

Movement (*i*.*e*. change of shape) of the SPs **4, 4',** shown in **FIG. 6** may be actuated by a single actuated joint **104** which movement is transmitted across the links of an SP. The position of the actuator may be anywhere between two links constituting an SP. There is preferably one actuated joint present in the pair of SPs. Alternatively, movement of the SPs **4, 4'** may be actuated by an actuator connected to one or both two revolute joints **116** and **117** of the base links **13, 7**. Torque applied to said base links would effect movement (*i.e*. change of shape) of the SPs **4, 4'.** In a specific embodiment, movement of the SPs **4, 4'** may be actuated by an actuator **119** (**FIG. 5**) connected to the revolute joints **116** and **117** of the base links **13, 7** through two pulleys **118.** The above mentioned timing belt **115,** would apply torque to both base links **13, 7.** The SPs **4, 4'** as shown in **FIG. 6** induces a circular motion of the effector link **11** in a plane parallel to the upper plate **102** of the base **2.**

Preferably, reversible actuators are used, to allow the structure to be manipulated by hand when they are switched off, in order to allow the surgeon to grasp the instrument and move it by hand for a moment, without having to disconnect it from the device. When the surgeon wants to use the remote control again, the actuators can be activated and the system used in robot mode.

The rectangles **6, 6'** retain a constant shape as mentioned above *i.e*. always rectangular, therefore, actuation of the main active structure only changes the position in space of the distal rectangle and not its shape.

The main active structure **100** also may also comprise several switches and encoders that send information about the angular position and the rate of motion of the effector link **11** to the central control unit of the device.

### Balancing mechanism

Either or both FPs **5** and **5'** may be equipped with a static balancing mechanism **121** that uses springs to compensate the weight of the mobile parts of the device, including the surgical instrument, the local zoom device, the passive adjustable arm, the end-effector and the mobile parts of the main active structure. The static balancing mechanism may comprise one or more springs connecting two adjacent links of an FP **5, 5'.** These static balancing mechanisms **121** prevent the instrument from losing its position in case of loss of current. They also make the manual manipulation of the structure easier and smoother, and allow the use of smaller actuators, which improves the safety in case of wrong manipulation or malfunction.

### Other components of the system

As shown in **FIG. 7**, the hybrid manual-robotic system may further comprise components in addition to the active main structure **100**. There may be a transmission means **200** that is linked to the main active structure, preferably via the effector link **11**. A local zoom device **300** may be attached to the transmission means **200.** A clamping mechanism **400** may be attached to the base of the active structure to mount the robotic device on the lateral table rail **110**. The hybrid manual-robotic system may be carried in a rolling base **500** and stored therein when it is not used. The rolling base **500** may comprise different peripheral components, including a central control unit that receives the surgeon's orders through the control device, computes the required motions of the robotic device, and sends output signals to actuators to produce the desired motion of the instrument held by the local zoom device.

### Transmission means

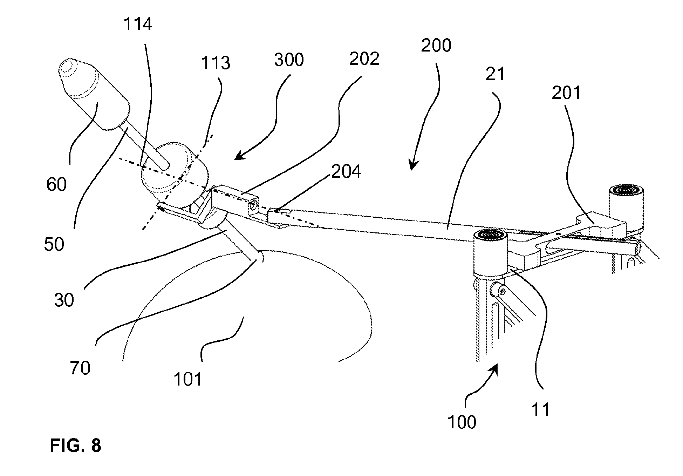

The transmission means **200** shown in **FIG. 8** connects the instrument (*e.g.* a local zoom device **300**) to the distal rectangle **6**, preferably the effector link **11** of the active main structure **100.** It is preferably passive *i.e*. not actuated. The transmission means may comprises an articulated arm **21** that can be rigidly locked, for transmitting movements of the main active structure over the distance of the articulated arm **21** to the instrument. It may comprise other main parts *e.g.* a fixing mechanism **201** to mount the articulated arm **21** onto the effector link **11** of the active main structure **100,** and a quick clamping mechanism **202** that grasps the instrument (*e.g.* local zoom device **300**). The transmission means **200** can be attached to the distal rectangle **6**, preferably the effector link **11** of the active main structure **100**, through the sterile bag. This construction allows the transmission means **200** to be temporarily withdrawn without compromising the sterility.

### Fixing mechanism and articulated arm

The articulated arm **21** may be made of several rigid bars which are articulated by lockable passive joints. The articulated arm **21** can thus be rigidly locked for transmitting movements of the main active structure over the distance of the articulated arm **21** to the instrument. It allows the surgeon to adjust the position of the second end of the articulated arm, where the local zoom device **300** is connected, above a stationary point **70** in the abdominal wall, once the active main structure **100** is already mounted on the rail table **110**. The articulated arm **21** may also be provided with fasteners to hold the camera and light cables.

The fixing mechanism **201** allows attachment of the articulated arm **21** to the distal rectangle **6,** preferably to the effector link **11,** so that movement of the active structure is transmitted to the instrument. The fixing mechanism **201** is generally one that can lock the position of the articulated arm **21** relative to the distal rectangle **6** or effector link **11** rigidly during use, but may permit adjustment of the articulated arm **21** relative to the distal rectangle **6** or effector link **11** when unlocked. Typically, the fixing mechanism **201** may allow articulated arm **21** to translate along its longitudinal axis relative to the distal rectangle **6** or effector link **11** when unlocked. Typically, the fixing mechanism **201** may allow articulated arm **21** to pivot parallel to the operating surface **150** relative to the distal rectangle **6** or effector link **11** when unlocked. Typically, the fixing mechanism **201** may allow articulated arm **21** to pivot around an axis parallel base links **7, 13**. The fixing mechanism **201** may also allow the articulated arm to be released from the active structure. The articulated arm **21** may comprise a longitudinal slot that fits into a reciprocating slot or hole present in the fixing mechanism **201** through which a screw passes that can be tightened to lock the articulated arm **21** or untightened to unlock the arm as required.

### Quick clamping mechanism

The transmission may further comprise a quick clamping mechanism **202** that couples the instrument to the distal end of the articulated arm **21.** The coupling is preferably a lockable revolute joint **204** configured to allow the quick clamping mechanism **202** to pivot parallel to the axis of the operating surface **150** relative to the articulated arm **21.** This axis of the joint **204** may be essentially parallel to the longitudinal axis of the base links **7, 13**. The revolute joint **204** is generally one that can lock the position of the quick clamping mechanism **202** relative to the articulated arm **21** rigidly during use, but may permit adjustment of the quick clamping mechanism **202** relative to the articulated arm **21** when unlocked. Typically, the revolute joint **204** allows quick clamping mechanism **202** to pivot parallel to the operating surface **150** relative to the articulated arm **21** when unlocked. The revolute joint **204** may also allow the quick clamping mechanism **202** to be released from the articulated arm **21.** The revolute joint **204** may comprise a screw that passes through both the quick clamping mechanism **202** and the articulated arm **21**, that can be tightened to lock the quick clamping mechanism **202** or untightened to unlock the mechanism as required. Alternatively, the quick clamping mechanism **202** can be attached to the articulated arm **21** through a safety mechanism that releases the arm **21** and allows it to swivel around the revolute joint if a large force is exerted on the arm **21**, to prevent any injury to the patient. An example of such a safety mechanism is made of a ball pressed by a spring against a spherical hole in the quick clamping mechanism **202**.

### Instrument

The instrument attaches to the distal end of the transmission means **200** preferably via the quick clamping mechanism **202**. The instrument may be permitted to swivel with respect to the quick clamping mechanism **202** by virtue of a universal joint or an equivalent thereof. For example, the instrument may be connected to the quick clamping mechanism **202** using two perpendicularly arranged passive revolute joints **113** and **114**. As mentioned herebefore, this arrangement allows the instrument to swivel around the stationary point **70**, for example, in the abdominal wall when the active main structure **100** is actuated.

Where the instrument is a laparoscope, a local zoom device **300**, illustrated in **FIG. 6**, may hold the surgical instrument, which local zoom device is attached to the quick clamping mechanism **202** using two perpendicularly arranged passive revolute joints **113** and **114**. The local zoom device **300** may be equipped with one or several actuators that can turn the laparoscope around its longitudinal axis, and advance it and move it back in the abdominal cavity **10**, through the cannula **30**. The device may also include an encoder system that can measure the displacement of the laparoscope **5** and send this information to the central control unit.

### Table Clamping mechanism

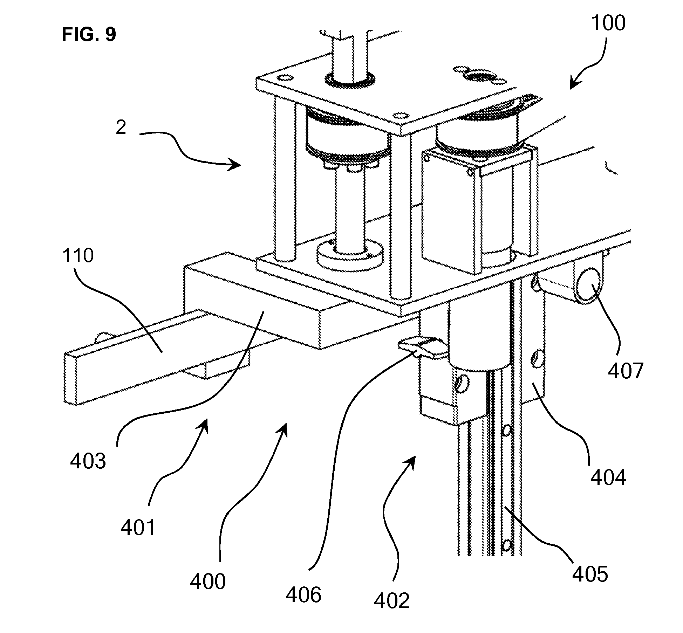

The hybrid manual-robotic system may be secured to the lateral table rail **110** by the clamping mechanism **400**. As shown in **FIG. 9**, the table clamping mechanism **400** may comprise a securing mechanism **401** and a sliding mechanism **402.**

The securing mechanism **401** is similar to a clamp, with typically a screw or a cam moved by a handle, that presses the lateral table rail **110** against the main rigid frame **403**.
The sliding mechanism **402** comprises a carrier **404**, attached to main rigid frame **403** of the securing mechanism **401**, and a linear rail **405** attached to the base **2** of the active main structure **100**. The linear rail **405** can slide into the carrier **404** to adjust the level of the robotic structure to the height of the patient's abdominal wall. The carrier **404** comprises a clamp, with typically a screw or a cam moved by a handle **406**, to lock the rail **405** into the carrier **404** in the desired position.

In another embodiment, the sliding mechanism **402** may also be actuated by an electrical motor that moves the rail **405** up and down in response to an input signal sent by the surgeon with the control device.

The table clamping mechanism **400** may also comprise a tilting mechanism **407,** placed between the linear rail **405** and the base **2** of the main active structure **100**. This tilting mechanism **407** allows the robotic structure to be tilted around a horizontal axis, in order to fit the workspace of the active main structure **100** to the abdominal workspace required for the instrument.

### In use

According to an aspect of the invention, when the procedures begins, a nurse or a technical assistant brings the rolling base next to the operating table, grasps the main active structure **100** and mounts it onto the lateral table rail, in a position that does not obstruct the patient or the surgical team, while the surgeon makes the incisions with the trocars and places the cannulas for the laparoscope and the surgical instruments. When the device is secured on the table and the abdominal cavity is inflated, its height is adjusted approximately to the level of the stationary point in the abdominal wall. It is then draped with the sterile bag, to guarantee the sterility of the operating field. The device is then turned on and placed in its reference position.

After that, the surgeon clamps the sterile passive adjustable arm on the end-effector of the main active structure. He places the laparoscope or the instrument that has to be manipulated by the robot in the sterile local zoom device and puts it into the cannula. He grasps the zoom device and makes sure that the instrument is parallel to the connecting bars of the articulated structure.

With the other hand, he grasps the extremity of the passive adjustable arm and brings int next to the zoom device in order to connect them together. Finally, he locks the joints of the articulated arm to make it become rigid, so that every movement of the main active structure is now reproduced by the instrument or the laparoscope around the stationary point in the abdominal wall.

## Claims

1. A hybrid manual-robotic system for supporting and moving a surgical instrument having active main structure (100) that comprises:
a) a base (2) for attachment to an operating table having an operating surface (150),
b) a hexahedral-shape frame (3), which shape is formed from
- a first (5, 5') and second (4, 4') pair of opposing parallelograms and
- opposing proximal (6') and distal (6) rectangles, which frame (3) is formed by at least seven links (7, 8, 9, 10, 11, 12, 13, 22, 23) connected by revolute joints (14, 15, 16, 17, 18, 19, 20, 26, 27, 109),
c) whereby a pair of parallel base links (7, 13) of the proximal rectangle (6') is coupled to the base (2) and are configured to lie essentially perpendicular to the plane of the operating surface (150),
d) whereby said revolute joints are configured to allow the hexahedral frame (3) to freely adopt a cube, or parallelepiped restricted by said perpendicular coupling, and
e) whereby the distal rectangle (6) is coupled through a transmission means (200) to said instrument.

2. A system according to claim 1, wherein at least one joint of the first (5, 5') pair of opposing parallelograms and at least one joint of the second (4, 4') pair of opposing parallelograms are actuated.

3. A system according to claim 1 or 2, wherein
- the first pair of parallelograms (5, 5') is defined as the pair each comprising one link that is a base link (7, 13) of the proximal rectangle (6'), and
- the first pair of parallelograms are connected by a link of the distal rectangle (6) that is defined as an effector link (11) to which the transmission arm is coupled.

4. A system according to claim 3, wherein said effector link (11) is an uppermost link of the proximal rectangle (6').

5. A system according to claim 4, wherein the length of the effector link (11) is greater than the length of the FPs links (8, 10, 12, 23) that are joined to the base links (7, 13).

6. A system according to any of claims 1 to 5, equipped with a static balancing mechanism (121) configured to passively maintain the position of the instrument after movement.

7. A system according to any of claims 1 to 6, wherein the transmission means (200) comprises an articulated arm (21) that can be rigidly locked for transmitting movements of the main active structure over the distance of the articulated arm (21) to the instrument.

8. A system according to claim 7, wherein the transmission means further comprises a fixing mechanism (201) that couples the distal rectangle to the articulated arm (21).

9. A system according to claim 8, wherein said coupling is a lockable joint configured to allow the articulated arm (21) to:
- pivot parallel to the axis of the operating surface (150) relative to the distal rectangle (6), and
- translate along at least part of its longitudinal axis relative to the distal rectangle (6).

10. A system according to any of claims 1 to 9, wherein the transmission means further comprises a quick clamping mechanism (202) that couples the instrument to the distal end of the articulated arm (21).

11. A system according to claim 10, wherein said coupling is a lockable revolute joint configured to allow the quick clamping mechanism (202) to pivot parallel to the axis of the operating surface (150) relative to the articulated arm (21).

12. A system according to claim 10 or 11, wherein the instrument is a laparoscope attached to the quick clamping mechanism (202) *via* a local zoom device (300).

13. A system according to any of claims 1 to 12, wherein the base comprises a table clamping mechanism (400) for attachment to a lateral table rail (110) of the operating surface.
